(19) Europäisches Patentamt
European Patent Office
Office européen des brevets

(11) EP 0 720 978 B1

(12) **FASCICULE DE BREVET EUROPEEN**

(45) Date de publication et mention de la délivrance du brevet:
**31.03.1999 Bulletin 1999/13**

(51) Int Cl.6: **C07C 67/29**, C07C 69/54, C07D 317/24, C07D 301/12, C07D 301/14, C07D 303/16

(21) Numéro de dépôt: **96102424.7**

(22) Date de dépôt: **04.07.1991**

(54) **Procédé d'ouverture de la fonction époxyde d'époxydes (méth)acryliques**

Verfahren zum Öffnen der Epoxyfunktion von (Meth)acrylepoxyden

Process to open the epoxyde function of (meth)acrylic epoxydes

(84) Etats contractants désignés:
**AT BE CH DE DK ES FR GB GR IT LI LU NL SE**

(30) Priorité: **06.07.1990 FR 9008607**

(43) Date de publication de la demande:
**10.07.1996 Bulletin 1996/28**

(62) Numéro(s) de document de la (des) demande(s) initiale(s) en application de l'article 76 CBE:
**91401849.4 / 0 468 840**

(73) Titulaire: **ELF ATOCHEM S.A.**
**92800 Puteaux, Hauts-de-Seine (FR)**

(72) Inventeurs:
- **Caubere, Paul**
  **54000 Nancy (FR)**
- **Fort, Yves**
  **54500 Vandoeuvre les Nancy (FR)**
- **Ortar, Agnès**
  **54800 Jarny (FR)**

(74) Mandataire: **Chaillot, Geneviève**
**Cabinet CHAILLOT,**
**16-20, avenue de L'Agent Sarre,**
**B.P. 74**
**92703 Colombes Cédex (FR)**

(56) Documents cités:
**FR-A- 1 329 386** **US-A- 3 575 925**

- **DATABASE WPI Week 8549 Derwent Publications Ltd., London, GB; AN 85-307983 XP002002472 & JP-A-60 215 650 (NIPPON OIL & FATS KK) , 29 Octobre 1985**
- **"ULLMANN'S ENCYCLOPEDIA OF IND. CHEM.", vol. A9, pp. 531-545, VCH VERLAGSGESELLSCHAFT GMBH, D-6940 Weinheim, 1987**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 720 978 B1

## Description

La présente invention concerne un procédé d'ouverture de la fonction époxyde d'époxydes (méth)acryliques.

FR-A-1 329 386 décrit un procédé d'hydratation sélective d'époxy-ester dans lequel on hydrate le fonction époxy tout en laissant intacte la liaison ester à l'aide d'un acide minéral, tel que l'acide sulfurique, nitrique ... ; parmi les esters envisagés figurent les esters d'acide acrylique ainsi que les esters des alcools tels que le glycidol et le 2,3-époxybutane. Plus précisément, JP 60-215 650 décrit l'hydratation du glycidyl acrylate en présence d'un catalyseur acide, tel que l'acide sulfurique, l'acide nitrique, une résine échangeuse d'ions de type sulfonique. US-A-3 575 925, dans le cadre spécifique de la préparation de composés polymérisables sous l'action d'ultraviolets, décrit la formation de composés insaturés par réaction d'un acide tel que l'acide benzoïque ou naphtoïque avec un réactif tel que l'acrylate de glycidyle en présence notamment de sels de métaux alcalins, tels que les acétates de métaux alcalins.

Le problème qui est à la base de la présente invention est de fournir des alternatives à ces procédés connus.

L'époxyde (méth)acrylique soumis au procédé de la présente invention est représenté par la formule générale :

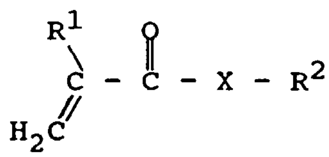

(I)

dans laquelle :

- X est choisi parmi les atomes d'oxygène et de soufre, le radical NH, les radicaux $NR^3$ dans lesquels $R^3$ est un groupe alkyle ayant de 1 à 12 atomes de carbone, et les radicaux oxyalkylène $O\text{-}(CH_2)_n$ dans lesquels n est un nombre entier allant de 3 à 16 ;
- $R^1$ est choisi parmi l'atome d'hydrogène et les radicaux alkyle ayant de 1 à 5 atomes de carbone ; et
- $R^2$ désigne une chaîne hydrocarbonée comprenant de 2 à 20 atomes de carbone, choisie parmi les chaînes alkyle linéaires ou ramifiées, cycloalkyle ou hétérocycloalkyle mono- ou polycycliques, alkylaryle, ladite chaîne hydrocarbonée comprenant un groupe oxiranne dans la chaîne ou en bout de chaîne dans le cas d'une chaîne alkyle ou alkylaryle, ou un groupe oxiranne exo- ou endocyclique dans le cas d'une chaîne cycloalkyle ou hétérocycloalkyle mono- ou polycyclique,
  $R^2$ pouvant également être le radical glycidyle, le radical 2-époxyéthylbicyclo[2.2.1]hept-5(6) -yle ou le radical époxydicyclopentényloxyéthyle.

Ce procédé est caractérisé par le fait que ledit époxyde (méth)acrylique est mis à réagir avec un composé choisi parmi les complexes d'éthérate et trifluorure de bore, les sels d'acides en présence de l'acide correspondant, comme l'acétate ou le propionate de sodium en présence respectivement d'acide acétique ou d'acide propionique, les halogénures de trialkyl- ou trialcoxy-silanes, notamment de triméthyl-, triéthyl-, triméthoxy- ou triéthoxysilane, plus particulièrement, le triméthylchlorosilane, et les cétones en présence de résines cationiques.

Comme époxydes (méth)acryliques de départ, on peut citer notamment des méthacrylates époxydés de formule :

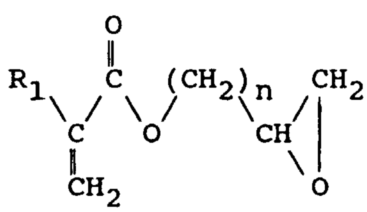

(Ia)

dans laquelle $R_1$ est choisi parmi l'atome d'hydrogène et le radical méthyle, et n est un nombre entier allant de 3 à 16, des (méth)acrylates de formule :

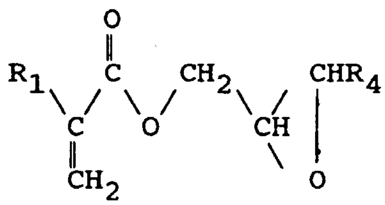

(Ib)

dans laquelle $R_1$ est choisi parmi l'atome d'hydrogène et le radical méthyle, et $R_4$ est choisi parmi les radicaux alkyle ayant de 1 à 12 atomes de carbone et les radicaux aryle ayant de 6 à 12 atomes de carbone,
ainsi que des (méth)acrylates époxydés de formules :

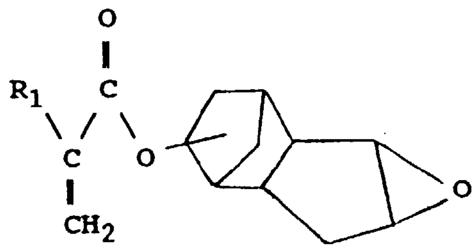

(Ic)

et

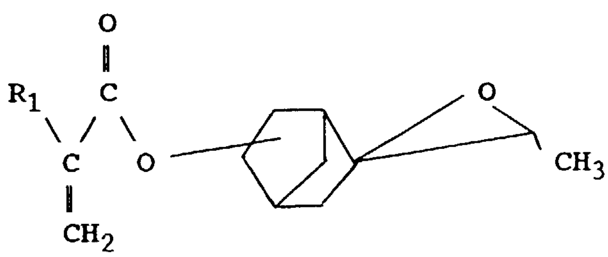

(Id)

dans lesquelles $R_1$ est choisi parmi l'atome d'hydrogène et le radical méthyle.

Comme exemples représentatifs, on peut citer les acrylates et méthacrylates de 4,5-époxypentyle, de 5,6-époxyhexyle, de 6,7-époxyheptyle, de 7,8-époxyoctyle, de 8,9-époxynonyle, de 9,10-époxydécyle et de 11,12-époxydodécyle. On peut également citer les acrylates et méthacrylates de 2,3-époxybutyle et de 3-phényl-2,3-époxypropyle. Les nouveaux (méth)acrylates époxydés de formules (Ic) et (Id) sont respectivement les acrylate et méthacrylate d'octahydro-2,5-méthano-2H-undéno[1,2-b]-oxyrényle et d'hydroxy-5(6)[époxy-2,1' éthyl]-2 bicyclo [2.2.1]heptane.

Selon la nature du composé réactif choisi, les conditions réactionnelles peuvent varier quelque peu et la nature du produit d'ouverture de la fonction époxyde varie également.

Lorsque le composé réactif est un complexe d'éthérate et de trifluorure de bore, celui-ci est utilisé en quantité sensiblement stoechiométrique par rapport à l'époxyde et la réaction a généralement lieu en présence d'un alcool saturé ayant de 1 à 4 atomes de carbone comme solvant réactif et à une température comprise entre -50°C et +30°C environ et pendant une durée pouvant atteindre jusqu'à 30 heures environ. Le produit d'ouverture formé est alors identifié comme un éther-alcool pouvant être représenté schématiquement par la formule :

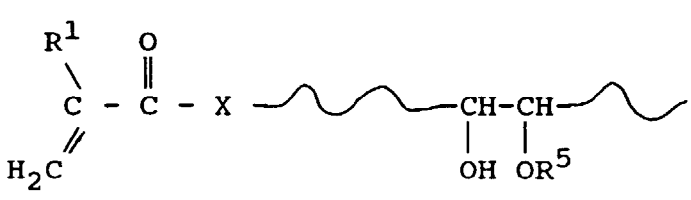

(II)

dans laquelle $R^5$ est un radical alkyle ayant de 1 à 4 atomes de carbone provenant de l'alcool solvant.

Lorsque le composé réactif est un sel d'acide, celui-ci est utilisé en excès dans un rapport de 2 à 10 moles environ par mole d'époxyde. La réaction est conduite en utilisant l'acide correspondant comme solvant à une température comprise entre 20 et 60°C environ et pendant une durée pouvant atteindre jusqu'à 72 heures environ. Le produit d'ouverture formé est alors identifié comme un acétate-alcool pouvant être représenté schématiquement par la formule :

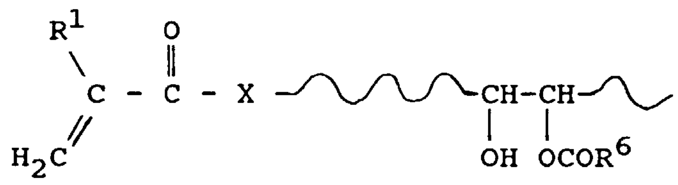
(III)

dans laquelle $R^6$ est un groupe alkyle ou aryle provenant du sel d'acide correspondant.
A titre d'exemple, on peut citer l'acétate de sodium comme composé réactif, en présence d'acide acétique, $R^6$ représentant alors $CH_3$.

Lorsque le composé réactif est un halogénure de trialkyl- ou trialcoxysilane, celui-ci est utilisé en quantité sensiblement stoechiométrique, 1 mole à 1,33 mole environ par rapport à 1 mole d'époxyde. La réaction est effectuée généralement en présence d'un catalyseur tel qu'une phosphine, la triphénylphosphine par exemple, ou un ammonium quaternaire, le bromure de tributylammonium par exemple, utilisé en quantité catalytique, 0,1 à 1% molaire environ par rapport à l'époxyde, et en présence d'un solvant ou d'un mélange de solvants, par exemple le chloroforme, le tétrachlorure de carbone, le chlorure de méthylène, le 1,2-dichloro-éthane. La quantité de solvant utilisée est d'environ 5 à 10 fois en poids celle de l'époxyde. La réaction a généralement lieu à une température comprise entre 0 et 25°C et pendant une durée comprise entre 10 minutes et 1 heure. Le produit d'ouverture formé est alors un mélange de chloro-éther silylé pouvant être représenté schématiquement par la formule :

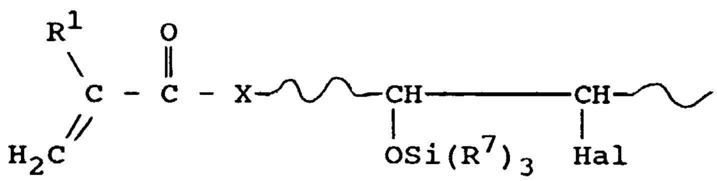
(IV)

dans laquelle :

- Hal est un halogénure, et
- $R^7$ est un groupe alkyle ou alcoxy provenant de l'halogénure de trialkyl- ou trialcoxysilane mis en jeu.

Il est à noter que ce composé conduit par hydrolyse à l'eau ou par l'humidité ambiante à la formation du chloro-alcool correspondant schématiquement représenté par la formule :

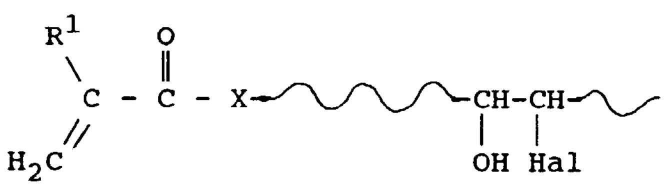
(IVa)

Lorsque le composé réactif est une cétone, la réaction a généralement lieu en présence d'au moins une résine cationique utilisée à raison de 20 à 50% environ en poids par rapport à l'époxyde (méth)acrylique, à une température comprise entre 10°C et 50°C environ et pendant une durée comprise entre 30 minutes et 3 heures environ. Comme cétone on peut utiliser notamment l'acétone, la méthyléthylcétone, l'acétophénone, la pentanone-3 ou la méthylisobutylcétone, dans un rapport de 1 à 2,5 moles environ pour 1 mole d'époxyde (méth)acrylique. Comme résine cationique on peut utiliser toute résine acide échangeuse d'ions telle que par exemple une résine sulfonée de faible granulométrie. Le produit d'ouverture formé est alors un dioxolane pouvant être représenté schématiquement par la formule :

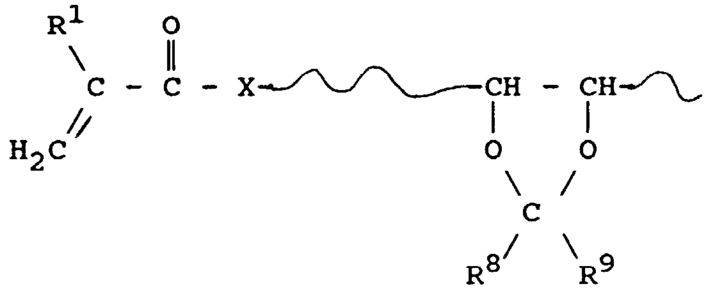 (V)

dans laquelle $R^8$ et $R^9$ sont des groupes alkyles ou aryles provenant de la cétone.

De préférence, les méthacrylates époxydes de formule (I) sont stabilisés, avant réaction, par l'addition d'une quantité efficace d'au moins un composé tel que l'hydroquinone, le monoéther méthylique de l'hydroquinone, la phénothiazine, la N,N-diéthylhydroxylamine, le nitrobenzène, le di-tertiobutylcatéchol, le p-anilinophénol, le phosphite de di-(2-éthylhexyl)octylphényle, le bleu de méthylène et leurs mélanges en toutes proportions. Par quantité efficace on entend une proportion généralement comprise entre 0,05 et 1% environ en poids du méthacrylate époxyde de formule (I).

Les diols, éther-alcools et dioxolanes qui dérivent des méthacrylates époxydés par ouverture de la fonction époxyde comme décrit précédemment, sont des composés possédant une faible odeur. Ils se polymérisent facilement et se copolymérisent aussi avec des monomères à insaturation éthylénique, tels que l'éthylène, ainsi que :

- un acrylate ou méthacrylate d'alkyle dont le groupe alkyle linéaire ou ramifié, le cas échéant substitué, par exemple par au moins un atome d'halogène comme le chlore ou le fluor et/ou par au moins un groupe hydroxyle, possède de 1 à 20 atomes de carbone,
- un acrylate ou méthacrylate d'aryle tel que le méthacrylate de benzyle,
- un hydrocarbure vinylaromatique tel que le styrène, le vinyltoluène, l'alphaméthylstyrène, le méthyl-4 styrène, le méthyl-3 styrène, le méthoxy-4 styrène, l'hydroxyméthyl-2 styrène, l'éthyl-4 styrène, l'éthoxy-4 styrène, le diméthyl-3,4 styrène, le chloro-2 styrène, le chloro-3 styrène, le chloro-4 méthyl-3 styrène, le tert.-butyl-3 styrène, le dichloro-2,4 styrène, le dichloro-2,6 styrène et le vinyl-1 naphtalène,
- un nitrile insaturé tel que l'acrylonitrile ou le méthacrylonitrile,
- un maléimide N-substitué tel que le N-éthylmaléimide, le N-isopropylmaléimide, le N-n-butylmaléimide, le N-isobutylmaléimide, le N-terbutylmaléimide, le N-n-octylmaléimide, le N-cyclohexylmaléimide, le N-benzylmaléimide et le N-phénylmaléimide,
- un anhydride d'acide dicarboxylique insaturé tel que l'anhydride maléique, l'anhydride itaconique, l'anhydride citraconique ou l'anhydride tétrahydrophtalique,
- l'acide acrylique ou méthacrylique,
- un acrylate ou méthacrylate de polyol comme les diacrylates et diméthacrylates de l'éthylèneglycol, du propylèneglycol, du 1,3-butanediol, du 1,4-butanediol, du 1,6-hexane-diol, du néopentylglycol, du 1,4-cyclohexane-diol, du 1,4-cyclo-hexanediméthanol, du 2,2,4-triméthyl-1,3-pentane-diol, du 2-éthyl-2-méthyl-1,3-propanediol, du 2,2-diéthyl-1,3-propanediol, du diéthylèneglycol, du dipropylèneglycol, du triéthylèneglycol, du tripropylèneglycol, du tétraéthylèneglycol, du tétrapropylèneglycol, du triméthyloléthane, du triméthylolpropane, du glycérol, du pentaérythritol, les triacrylates et triméthacrylates du triméthyloléthane, du triméthylolpropane, du glycérol, du pentaérythritol, les tétraacrylates et tétraméthacrylates du pentaérythritol, les di(méth)-acrylates à hexa(méth)acrylates du dipentaérythritol, les poly(méth)acrylates de polyols mono- ou polyéthoxylés ou mono- ou polypropoxylés tels le triacrylate et le triméthacrylate du triméthylolpropane triéthoxylé, du triméthylolpropane tripropoxylé ; le triacrylate et le triméthacrylate du glycérol tripropoxylé ; le triacrylate, le triméthacrylate, le tétraacrylate et le tétraméthacrylate du pentaérythritol tétraéthoxylé,
- un acrylamide ou méthacrylamide, un acrylate ou méthacrylate de dialkylaminoalkyle et leurs sels quaternaires,
- l'acrylate et le méthacrylate de (norbornyloxy-2)-2 éthyle et de (diméthanodécahydronaphtyloxy-2)-2 éthyle, et
- des oxazolidones acryliques et méthacryliques choisies parmi celles de formule :

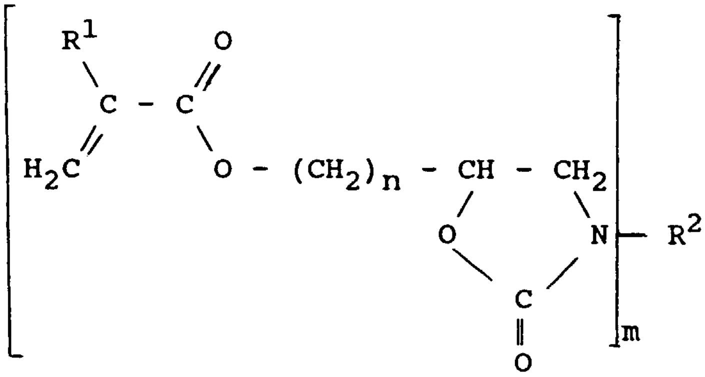

(VI)

et celles de formule :

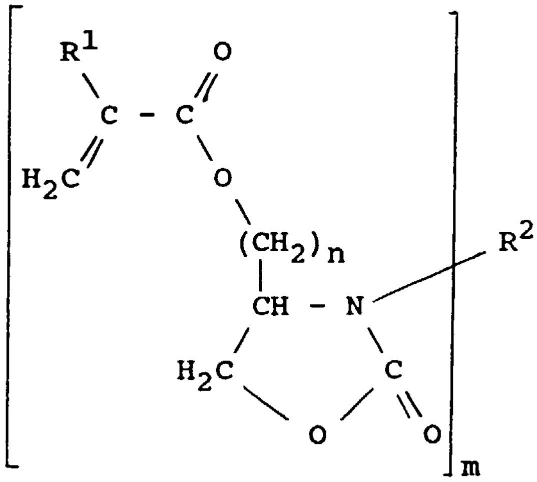

(VII)

formules dans lesquelles :

- $R^1$ est choisi parmi l'atome d'hydrogène et le radical méthyle,
- n est un nombre entier allant de 1 à 12,
- m est un nombre entier allant de 1 à 3, et
- $R^2$ est un radical hydrocarboné, alkyle linéaire, ramifié ou cyclique ou bien aromatique, possédant de 5 à 12 atomes de carbone,

lesdites oxazolidones pouvant être obtenues par réaction, entre 30°C et 90°C, d'un composé portant une fonction (méth)acrylique avec un composé portant au moins une fonction isocyanate,

- des composés acryliques et méthacryliques choisis parmi ceux de formule :

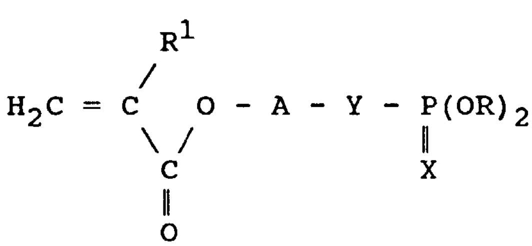

(VIII)

dans laquelle :

- $R^1$ est choisi parmi l'atome d'hydrogène et le radical méthyle,
- A est choisi parmi les radicaux $(CH_2)_n$ pour lesquels n est un nombre entier de 2 à 12 et le radical $-(CH_2CH_2O)_d-CH_2CH_2-$, d étant un nombre entier allant de 1 à 20,
- X est choisi parmi les atomes de soufre et d'oxygène,
- Y est choisi parmi les atomes de soufre et d'oxygène,
  avec la condition que X est un atome de soufre et Y est un atome d'oxygène lorsque A est le radical $-(CH_2CH_2O)_d-CH_2CH_2-$, et
- R est choisi parmi les radicaux alkyle ayant de 1 à 20 atomes de carbone et les groupes $-(CH_2)_pSR^3$ dans lesquels p est un nombre entier allant de 3 à 12 et $R^3$ est un radical alkyle ayant de 1 à 20 atomes de carbone,

ceux de formule :

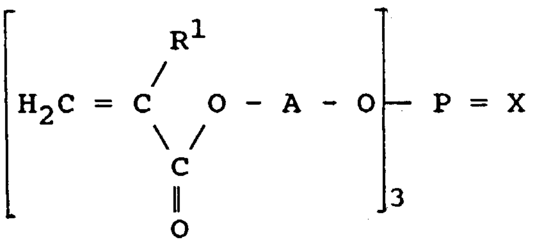

(IX)

dans laquelle :

- $R^1$ est choisi parmi l'atome d'hydrogène et le radical méthyle,
- A est choisi parmi les radicaux $(CH_2)_n$ pour lesquels n est un nombre entier de 2 à 12 et le radical $-(CH_2CH_2O)_d-CH_2CH_2-$, d étant un nombre entier allant de 1 à 20, et
- X est choisi parmi les atomes de soufre et d'oxygène,

et ceux de formule :

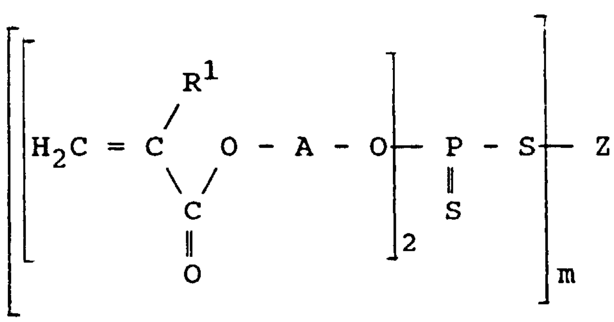

(X)

dans laquelle :

- $R^1$ est choisi parmi l'atome d'hydrogène et le radical méthyle,
- A est choisi parmi les radicaux $(CH_2)_n$ pour lesquels n est un nombre entier de 2 à 12,
- m est un nombre entier allant de 1 à 3, et
- Z est choisi parmi l'atome d'hydrogène, les radicaux $R^2QH$, $R^2$ étant un radical alkyle ayant de 2 à 12 atomes de carbone et Q étant choisi parmi les atomes d'oxygène et de soufre, et les atomes des métaux des groupes IA, IIA, IIIA, IB, IIB, VIB, VIIB et VIII de la Classification Périodique, avec la condition que Z est choisi parmi l'atome d'hydrogène et les radicaux $R^2OH$ lorsque m = 1 et que m est la valence de Z lorsque Z est un métal.
  De tels composés peuvent être préparés par réaction d'un composé acrylique ou méthacrylique de formule :

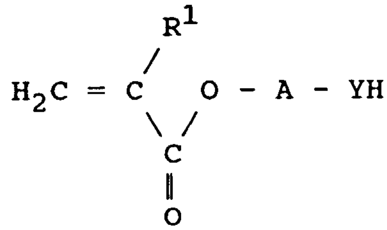

(XI)

dans laquelle $R^1$, A et Y ont les mêmes significations que dans la formule (X), avec un composé pentavalent du phosphore, celui-ci pouvant être par exemple un composé de formule $PXT_3$ dans laquelle X a la même signification que dans la formule (X) et T désigne un atome d'halogène, ou bien un composé phosphoré de formule :

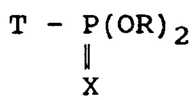

(XII)

dans laquelle R et X ont les mêmes significations que dans la formule (I) et T désigne un atome d'halogène, ou bien le pentasulfure $P_2S_5$,

- des composés acryliques et méthacryliques choisis parmi ceux de formule :

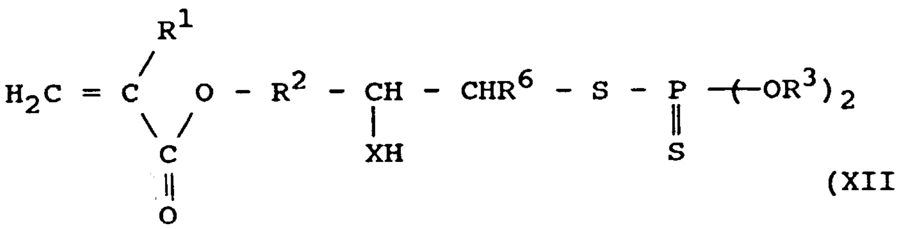

(XIII)

et ceux de formule :

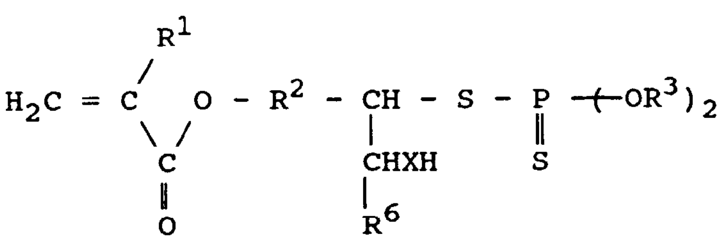

(XIV)

formules dans lesquelles :

- $R^1$ est choisi parmi l'atome d'hydrogène et le radical méthyle,
- X est un hétéroatome choisi parmi l'oxygène et le soufre,
- $R^2$ est choisi parmi les groupes alkylènes linéaires ou ramifiés, cycloalkylènes et hétérocycloalkylènes mono- ou polycycliques, alkylarylènes et arylalkylènes comprenant de 1 à 12 atomes de carbone,
- $R^6$ est choisi parmi l'atome d'hydrogène et les radicaux alkyles et aryles ayant de 1 à 12 atomes de carbone, et
- $R^3$ est choisi parmi les radicaux alkyles et aryles ayant de 1 à 20 atomes de carbone, les groupes $-(CH_2)_pSR^4$ dans lesquels p est un nombre entier allant de 2 à 12 et $R^4$ est un radical alkyle ayant de 1 à 20 atomes de carbone ou bien un groupe cycloalkyle mono- ou poly-cyclique ayant de 4 à 10 atomes de carbone, chaque

cycle dudit groupe comprenant de 4 à 6 chaînons, et les groupes

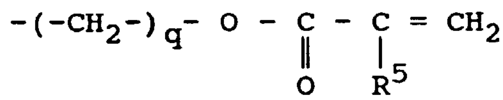

dans lesquels q est un nombre entier allant de 2 à 12 et $R^5$ est choisi parmi l'atome d'hydrogène et le radical méthyle. De tels composés peuvent être préparés par réaction d'un époxyde ou épisulfure acrylique ou méthacrylique de formule :

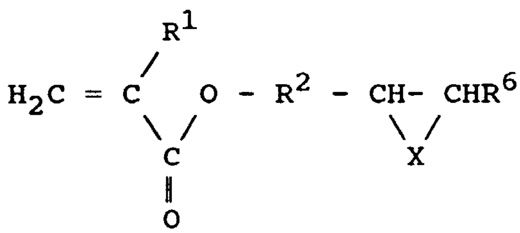

(XV)

dans laquelle $R^1$, $R^2$, $R^6$ et X ont les mêmes significations que dans la formule (XIII), avec un composé thiophosphoré de formule :

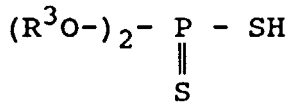

(XVI) .

De tels polymères et copolymères sont obtenus en (co)polymérisant au moins un composé acrylique soufré selon l'invention et, le cas échéant, au moins un comonomère copolymérisable, tel que défini précédemment, en présence d'au moins un initiateur de radicaux libres tel qu'un peroxyde, un hydroperoxyde ou un composé diazo. La (co) polymérisation est généralement effectuée à une température comprise entre 50°C et 120°C environ et en utilisant l'un des monomères comme solvant. Elle peut également s'effectuer en émulsion dans l'eau, à une température comprise entre 50°C environ et 100°C, en présence d'au moins un agent tensio-actif.

Lorsque le comonomère est l'éthylène, on peut utiliser une température de 140° à 300°C environ et une pression de 1000 à 3000 bars environ. Lorsque le comonomère est un hydrocarbure vinylaromatique, on peut utiliser une température de 80° à 200°C environ.

Les produits d'ouverture de la fonction époxyde, les éther-alcools de formule (II) et les dioxolanes de formule (V), en raison de leur faible odeur et de leur basse viscosité, pourront trouver des applications en tant qu'agents de modification dans les domaines des encres, des adhésifs, des peintures, des revêtements et des résines.

Les exemples suivants sont donnés à titre illustratif et non limitatif de la présente invention.

EXEMPLE 1

On place dans un réacteur 48 millimoles d'acétate de sodium et 50 ml d'acide acétique glacial. Une solution de 10 millimoles de méthacrylate de glycidyle dans 5 ml de chlorure de méthylène est ajoutée goutte à goutte. Le mélange obtenu est agité 2 jours à température ambiante. A la fin de cette période, 20 ml d'eau sont ajoutés et la phase aqueuse est extraite au chlorure de méthylène. La phase organique est lavée par une solution saturée de $NaHCO_3$. Après élimination des solvants, on soumet les produits organiques de la réaction à une chromatographie sur colonne de silice, en utilisant des éluants de polarités croissantes : mélange de 85% d'éther de pétrole et de 15% d'acétate d'éthyle, puis 50% d'éther de pétrole et 50% d'acétate d'éthyle. La réaction conduit avec un rendement de 44% à un mélange des acétates-alcools isomères de formules :

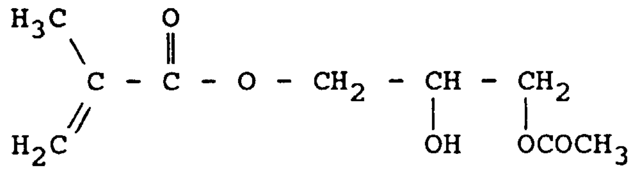

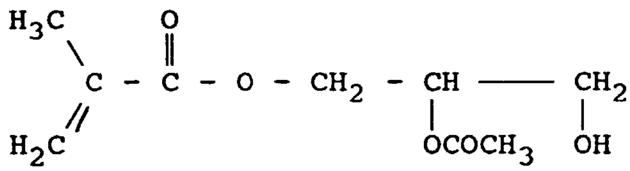

Le méthacrylate d'hydroxy-2 acétoxy-3 propyle a été caractérisé par :

- spectroscopie infrarouge : le spectre obtenu montre des bandes caractéristiques à 3478 $cm^{-1}$, 2960 $cm^{-1}$, 1739 $cm^{-1}$, 1723 $cm^{-1}$, 1634 $cm^{-1}$, 1454 $cm^{-1}$, 1297 $cm^{-1}$, 1165 $cm^{-1}$ et 945 $cm^{-1}$.
- résonance magnétique nucléaire du proton : déplacements chimiques à 6,05 ppm (m, 1H), 5,5 ppm (m, 1H), 4,3-3,4 ppm (m, 6H dont 1 O$\underline{H}$), 2,0 ppm (m, 3H) et 1,9 ppm (m, 3H).
- résonance magnétique nucléaire du carbone 13 : déplacements chimiques à 170,6 ppm (C=O), 166,7 ppm (C=O), 135,4 ppm (>C=), 125,7 ppm ($CH_2$=), 67,2 ppm ($\underline{C}$HOH), 64,9 ppm (O$\underline{C}H_2$), 62,2 ppm ($\underline{C}H_2$OCO), 20,2 ppm ($\underline{C}H_3$CO) et 17,7 ppm ($\underline{C}H_3$).

EXEMPLE 2

Dans un réacteur, on introduit 20 millimoles de méthacrylate de glycidyle et 25,56 g de chloroforme et 20,5 mg de triphénylphosphine. A la solution obtenue, 20 millimoles de chlorure de triméthylsilane sont additionnées en maintenant la température du milieu réactionnel en dessous de 25°C. Après 30 minutes, le solvant est évaporé et les composés organiques sont soumis à une chromatographie sur colonne d'alumine, en utilisant des éluants de polarités croissantes : mélange de 95% d'éther de pétrole et de 5% d'acétate d'éthyle puis 70% d'éther de pétrole et de 30% d'acétate d'éthyle. La réaction conduit avec un rendement de 33% de chloro-éther silylé et un rendement de 55% de chloro-alcool de formules respectives :

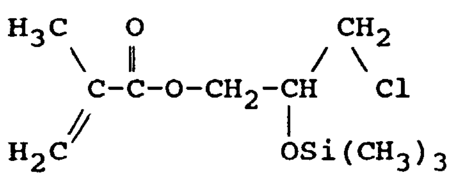 et 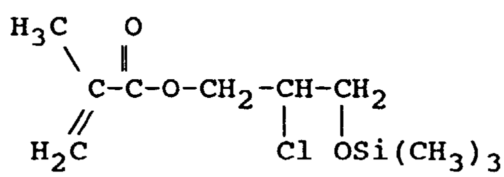

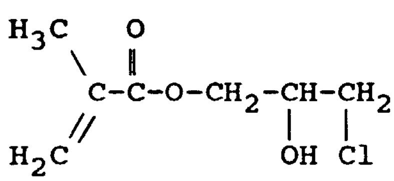 et 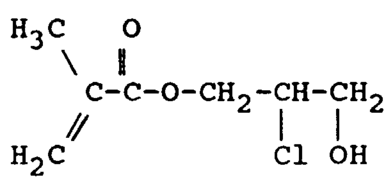

Les chloro-éthers silylés sont caractérisés par :

- spectroscopie infrarouge : le spectre obtenu montre des bandes caractéristiques à 2960 $cm^{-1}$, 1727 $cm^{-1}$, 1640 $cm^{-1}$, 1454 $cm^{-1}$, 1328 $cm^{-1}$, 1296 $cm^{-1}$, 1253 $cm^{-1}$ et 944 $cm^{-1}$.

- résonance magnétique nucléaire du proton : déplacements chimiques à 6,1 ppm (m, 1H), 5,55 ppm (m, 1H), 4,3-3,4 ppm (m, 5H), 2,0 ppm (m, 3H) et 0,2 ppm (s, 9H).

Les chloro-alcools sont caractérisés par :

- résonance magnétique nucléaire du proton : déplacements chimiques à 6,1 ppm (m, 1H), 5,5 ppm (m, 1H), 4,3-3,4 ppm (m, 6H dont 1 OH) et 2,0 ppm (m, 3H).

EXEMPLE 3

Dans un réacteur maintenu à -30°C et contenant 15 ml de méthanol on introduit successivement 10 millimoles de méthacrylate de glycidyle puis 10 millimoles d'éthérate de trifluorure de bore à 48% de $BF_3$ fraîchement distillé. On laisse ce mélange pendant 2 heures à -30°C sous agitation puis 12 heures à 25°C. On récupère alors, avec un rendement de 95% par rapport au méthacrylate de glycidyle, un mélange des méthacrylates isomères de formules :

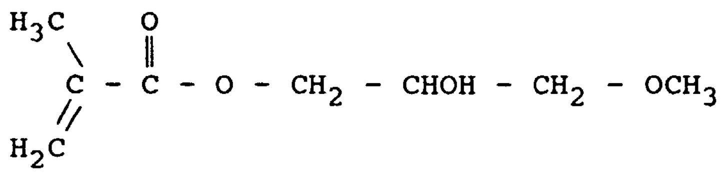

et

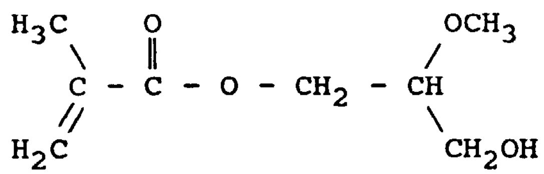

Le méthacrylate d'hydroxy-2-méthoxy-3 propyle a été caractérisé par

- spectrophotométrie infrarouge : le spectre obtenu (figure 1) montre des bandes caractéristiques à 3454, 2929, 1722, 1639, 1453, 1321, 1297 et 1171 $cm^{-1}$.
- résonance magnétique nucléaire du carbone 13 et du proton : les déplacements chimiques exprimés en ppm sont indiqués dans le tableau I ci-après.

EXEMPLE 4

Le processus opératoire de l'Exemple 3 est reproduit en remplaçant le méthacrylate de glycidyle par le méthacrylate de crotyle. On récupère avec un rendement de 91% un mélange des méthacrylates isomères de formules

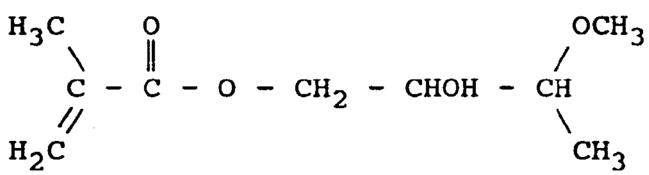

et

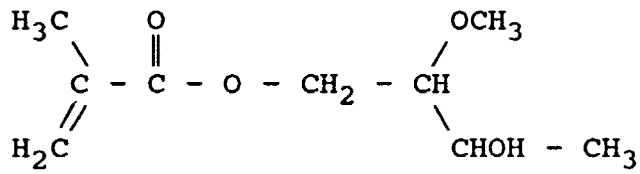

Le méthacrylate d'hydroxy-2-méthoxy-3 butyle a été caractérisé par :

- spectrophotométrie infrarouge : le spectre obtenu (figure 2) montre des bandes caractéristiques à 3460, 2933, 1722, 1638, 1453, 1322, 1298 et 1170 $cm^{-1}$.
- résonance magnétique nucléaire du carbone 13 et du proton : les déplacements chimiques exprimés en ppm sont indiqués dans le tableau I ci-après.

EXEMPLE 5

Le processus opératoire de l'exemple 3 est reproduit en remplaçant le méthacrylate de glycidyle par le méthacrylate d'époxydicyclopenténylоxyéthyle. On récupère avec un rendement de 90% un mélange de méthacrylates isomères de formule

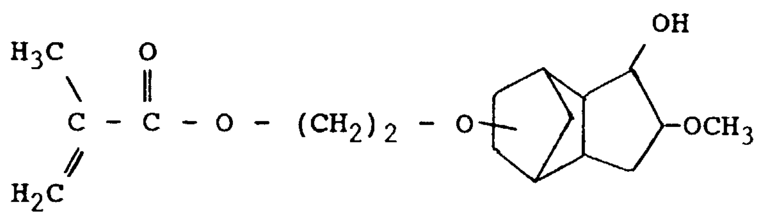

qui est caractérisé par :

- spectrophotométrie infrarouge : le spectre obtenu (figure 3) montre des bandes caractéristiques à 3435, 2949, 1722, 1638, 1452, 1321, 1296 et 1168 $cm^{-1}$.
- résonance magnétique nucléaire du carbone 13 et du proton : les déplacements chimiques exprimés en ppm sont indiqués dans le tableau I ci-après.

TABLEAU I

| Exemple | 3 | 4 | 5 |
|---|---|---|---|
| RMN $^{13}C$ | | | |
| δ (CO) | 166,9 | 167,4 | 166,9 |
| δ (C=) | 135,5 | 135,9 | 135,7 |
| δ ($CH_2$=) | 125,6 | 125,7 | 125,3 |
| δ ($CH_2$-O-$CH_3$) | 73,2 | 77,4 | 86,1-89,4 |
| δ (CHOH) | 67,9 | 71,6 | 76,3 |
| δ ($CH_2$-O) | 65,3 | 65,9 | 63,6-65,6-81,2 |
| δ ($OCH_3$) | 58,6 | 56,3 | 57,1-57,6 |
| δ ($CH_3$) | 17,8 | 14,2-18,0 | 17,8 |
| RMN $^1H$ | | | |
| δ ($CH_2$=) | 6,1 (m,1H) 5,5 (m,1H) | 6,05 (m,1H) 5,55 (m,1H) | 6,1 (m,1H) 5,5 (m,1H) |
| δ ($CH_2$) | 3,2-4,3 (m,8H,OH) | 3,1-4,2 (m,7H,OH) | 3,2-3,8 (m,8H,OH) |
| δ ($CH_3$) | 1,9 (m,3H) | 1,9 (m,3H) | 0,9-2,4 (m,13H) |
| δ ($CH_3$) | | 1,1 (m,3H) | |
| δ ($OCH_2$) | | | 4,1-4,4 (m,2H) |

EXEMPLE 6

Le mode opératoire de l'exemple 3 est reproduit en remplaçant le méthacrylate de glycidyle par l'acrylate d'époxydicyclopenténylloxyéthyle. On récupère avec un rendement de 92% un mélange d'acrylates isomères de formule

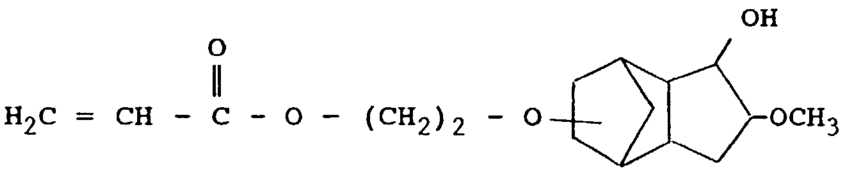

qui est caractérisé par :

- spectrophotométrie infrarouge : le spectre obtenu (figure 4) montre des bandes caractéristiques à 3436, 2955, 1728, 1637, 1450, 1297 et 1194 $cm^{-1}$.
- résonance magnétique nucléaire du carbone 13 et du proton : les déplacements chimiques exprimés en ppm sont indiqués dans le tableau II ci-après.

EXEMPLE 7

On place dans un réacteur 20 millimoles de méthacrylate de glycidyle, 40 millimoles de méthyléthylcétone et 1,02 gramme d'une résine échangeuse d'ions, de granulométrie 20 à 50 mesh, commercialisée sous l'appellation AMBERLIST 15. Le mélange est agité à 25°C pendant 2 heures. La réaction conduit avec un rendement de 40% à la formation de dioxolane de formule

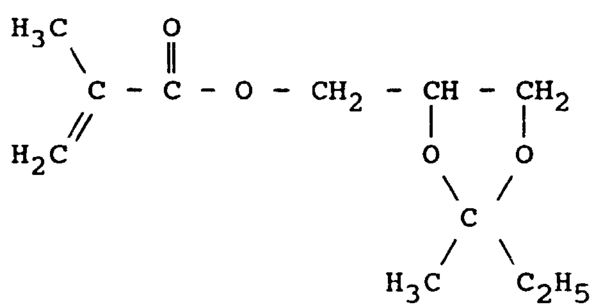

qui est caractérisé par :

- spectrophotométrie infrarouge : le spectre obtenu (figure 5) montre des bandes caractéristiques à 2982, 1724, 1639, 1453 et 1166 $cm^{-1}$.
- résonance magnétique nucléaire du carbone 13 et du proton : les déplacements chimiques exprimés en ppm sont indiqués dans le tableau II ci-après.

EXEMPLE 8

Le dioxolane obtenu à l'Exemple 7 est placé dans un réacteur avec 5 ml d'acide sulfurique à 10% et 10 ml d'éther diéthylique. Le mélange est agité à 25°C pendant 14 heures. La réaction conduit avec un rendement de 61% à la formation du diol de formule

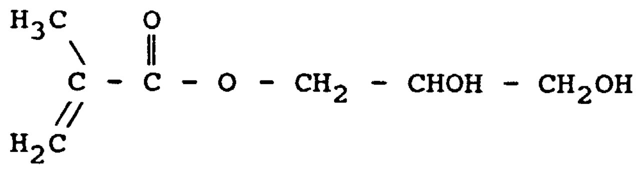

EXEMPLE 9

On reproduit le mode opératoire de l'Exemple 7 en remplaçant le méthacrylate de glycidyle par le méthacrylate d'époxycrotyle. La réaction conduit avec un rendement de 43% à la formation de dioxolane de formule

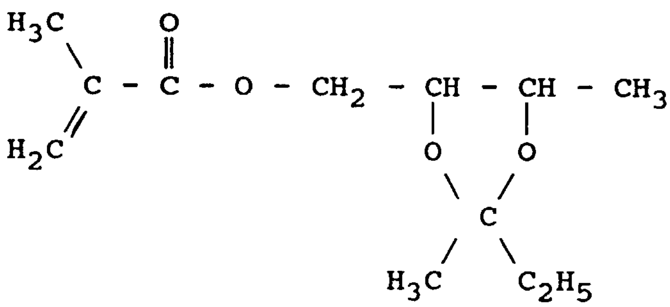

qui est caractérisé par :

- spectrophotométrie infrarouge : le spectre obtenu (figure 6) montre des bandes caractéristiques à 2982, 1723, 1639, 1453 et 1165 $cm^{-1}$.
- résonance magnétique nucléaire du carbone 13 et du proton : les déplacements chimiques exprimés en ppm sont indiqués dans le tableau II ci-après.

EXEMPLE 10

Le dioxolane obtenu à l'Exemple 9 est soumis aux conditions opératoires de l'Exemple 8. La réaction conduit avec un rendement de 77% à la formation du diol de formule :

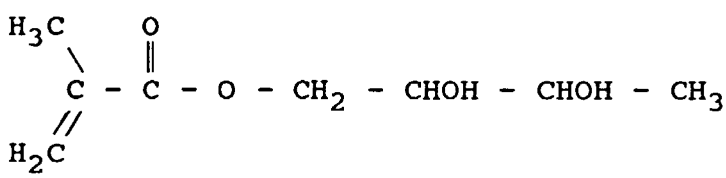

TABLEAU II

| Exemple | 24 | 25 | 27 |
|---|---|---|---|
| RMN $^{13}C$ | | | |
| δ (CO) | 165,1 | 166,4 | 166,2 |
| δ ($CH_2$=) | 130,1 | 125,4 | 125,3 |
| δ (C=) | 127,4 | 135,5 | 136,8 |
| δ ($CHOCH_3$) | 85,6-88,7 | | |
| δ ($CH_2$-O) | 63,0-65,0-81,5 | 64,0-73,6 | 62,8-77,8 |
| δ (CHOH) | 75,6 | | |

TABLEAU II (suite)

| Exemple | 24 | 25 | 27 |
|---|---|---|---|
| RMN $^{13}C$ | | | |
| δ ($OCH_3$) | 56,8 | | |
| δ ($CH_2$) | 28,9-49,9 | 31,1-32,0 | 29,3-33,0 |
| δ (C-O) | | 111,1 | 109,5-111,3 |
| δ ($CH_3$) | | 7,6-17,8-23,6 | 8-15-18-24 |
| RMN $^{1}H$ | | | |
| δ ($CH_2$=) | 5,6-6,7 (m,3H) | 6,0 (m,1H) | 6,0 (m,1H) |
| | | 5,5 (m,1H) | 5,45 (m,1H) |
| δ ($OCH_2$) | 4,0-4,4 (m,2H) | 3,5-4,4 (m,5H) | 3,5-4,5 (m,4H) |
| δ ($CH_3$) | 3,1-3,8 (m,8H) | 1,9 (m,3H) | 1,9 (m,3H) |
| δ (OH) | 2,7 (m,1H,OH) | | |
| δ ($CH_2$) | 0,9-2,4 (m,10H) | 0,7-1,8 (m,8H) | 0,7-1,8 (m,11H) |

## Revendications

**1.** Procédé d'ouverture de la fonction époxyde d'un époxyde (méth)acrylique représenté par la formule générale :

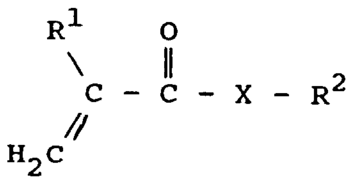

(I)

dans laquelle :

- X est choisi parmi les atomes d'oxygène et de soufre, le radical NH, les radicaux $NR^3$ dans lesquels $R^3$ est un groupe alkyle ayant de 1 à 12 atomes de carbone, et les radicaux oxyalkylène $O-(CH_2)_n$ dans lesquels n est un nombre entier allant de 3 à 16 ;
- $R^1$ est choisi parmi l'atome d'hydrogène et les radicaux alkyle ayant de 1 à 5 atomes de carbone ; et
- $R^2$ désigne une chaîne hydrocarbonée comprenant de 2 à 20 atomes de carbone, choisie parmi les chaînes alkyle linéaires ou ramifiées, cycloalkyle ou hétérocycloalkyle mono- ou polycycliques, alkylaryle, ladite chaîne hydrocarbonée comprenant un groupe oxiranne dans la chaîne ou en bout de chaîne dans le cas d'une chaîne alkyle ou alkylaryle, ou un groupe oxiranne exo- ou endocyclique dans le cas d'une chaîne cycloalkyle ou hétérocycloalkyle mono- ou polycyclique,
  $R^2$ pouvant également être le radical glycidyle, le radical 2-époxyéthylbicyclo[2.2.1]hept-5(6)-yl ou le radical époxydicyclopentényloxyéthyle,

caractérisé par le fait que ledit époxyde (méth)acrylique est mis à réagir avec un composé choisi parmi :

- les complexes d'éthérate et trifluorure de bore, utilisés en quantité stoechiométrique par rapport à l'époxyde ;
- les sels d'acides en présence de l'acide correspondant, utilisés en excès dans un rapport de 2 à 10 moles par mole d'époxyde ;
- les halogénures de trialkyl- ou trialcoxy-silanes, utilisés en quantité sensiblement stoechiométrique par rapport à l'époxyde ; et
- les cétones en présence de résines cationiques.

**2.** Procédé selon la revendication 1, le composé réactif étant un complexe d'éthérate et de trifluorure de bore, caractérisé par le fait que la réaction a lieu en présence d'un alcool saturé ayant de 1 à 4 atomes de carbone et à une température comprise entre -50°C et +30°C.

3. Procédé selon la revendication 1, le composé réactif étant un sel d'acide, caractérisé par le fait que l'on conduit la réaction en utilisant l'acide correspondant comme solvant à une température comprise entre 20 et 60°C.

4. Procédé selon la revendication 1, le composé réactif étant un sel d'acide, caractérisé par le fait que ledit sel est l'acétate ou le propionate de sodium, l'acide correspondant étant respectivement l'acide acétique ou l'acide propionique.

5. Procédé selon la revendication 1, le composé réactif étant un halogénure de trialkyl- ou trialcoxysilane, caractérisé par le fait que celui-ci est utilisé à raison d'1 mole à 1,33 mole par rapport à 1 mole d'époxyde, la réaction étant effectuée en présence d'un catalyseur tel qu'une phosphine et d'au moins un solvant, à une température de 0°C à 25°C.

6. Procédé selon la revendication 1, le composé réactif étant une cétone, caractérisé par le fait que la réaction a lieu à une température comprise entre 10°C et 50°C et en présence d'au moins une résine cationique à raison de 20 à 50% en poids par rapport à l'époxyde (méth)acrylique.

**Patentansprüche**

1. Verfahren zum Öffnen der Epoxygruppe einer Epoxy(meth)-acrylverbindung der folgenden allgemeinen Formel:

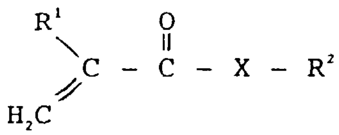

(I),

worin:

- X ausgewählt ist unter Sauerstoff und Schwefel, der Gruppe NH, den Gruppen $NR^3$, worin $R^3$ eine Alkylgruppe mit 1 bis 12 Kohlenstoffatomen bedeutet, und Alkoxygruppen $O\text{-}(CH_2)_n$, worin n eine ganze Zahl im Bereich von 3 bis 16 bedeutet;
- $R^1$ ausgewählt ist unter Wasserstoff und Alkylgruppen mit 1 bis 5 Kohlenstoffatomen; und
- $R^2$ eine Kohlenwasserstoffkette bedeutet, die 2 bis 20 Kohlenstoffatome enthält und die unter geradkettigen oder verzweigten Alkylgruppen, monocyclischen oder polycyclischen Cycloalkylgruppen oder Heterocycloalkylgruppen und Alkylarylgruppen ausgewählt ist, wobei die Kohlenwasserstoffgruppe in der Kette oder am Ende der Kette eine Oxirangruppe enthält, wenn es sich um eine Alkylgruppe oder eine Alkylarylgruppe handelt, oder die Kohlenwasserstoffgruppe eine exocyclische oder endocyclische Oxirangruppe enthält, wenn es sich um eine monocyclische oder polycyclische Cycloalkylgruppe oder Heterocycloalkylgruppe handelt, wobei $R^2$ ferner Glycidyl, 2-Epoxyethylbicyclo[2.2.1]-hept-5(6)-yl oder Epoxydicyclopentenyloxyethyl sein kann,

dadurch gekennzeichnet, daß die Epoxy(meth)acrylverbindung mit einer Verbindung umgesetzt wird, die ausgewählt ist unter:

- Etheratkomplexen mit Bortrifluorid, die bezogen auf das Epoxid in einer stöchiometrischen Menge verwendet werden;
- Säuresalzen in Gegenwart der entsprechenden Säure, die im Überschuß in einem Verhältnis von 2 bis 10 mol pro Mol Epoxid verwendet werden;
- Trialkylsilanhalogeniden oder Trialkoxysilanhalogeniden, die bezogen auf das Epoxid in einer im wesentlichen stöchiometrischen Menge verwendet werden, und
- Ketonen in Gegenwart kationischer Harze.

2. Verfahren nach Anspruch 1, wobei die reaktive Verbindung ein Etheratkomplex mit Bortrifluorid ist, dadurch gekennzeichnet, daß die Umsetzung in Gegenwart eines gesättigten Alkohols mit 1 bis 4 Kohlenstoffatomen und bei einer Temperatur im Bereich von -50 °C bis +30 °C erfolgt.

**3.** Verfahren nach Anspruch 1, wobei die reaktive Verbindung ein Säuresalz ist, dadurch gekennzeichnet, daß die Umsetzung bei einer Temperatur im Bereich von 20 bis 60 °C unter Verwendung der entsprechenden Säure als Lösungsmittel durchgeführt wird.

**4.** Verfahren nach Anspruch 1, wobei die reaktive Verbindung ein Säuresalz ist, dadurch gekennzeichnet, daß es sich bei dem Salz um Natriumacetat oder Natriumpropionat handelt, wobei die entsprechende Säure Essigsäure bzw. Propionsäure ist.

**5.** Verfahren nach Anspruch 1, wobei die reaktive Verbindung ein Trialkylsilanhalogenid oder ein Trialkoxysilanhalogenid ist, dadurch gekennzeichnet, daß das Trialkylsilanhalogenid oder das Trialkoxysilanhalogenid in einem Mengenanteil von 1 mol bis 1,33 mol, bezogen auf 1 mol Epoxid, verwendet wird, wobei die Umsetzung bei einer Temperatur im Bereich von 0 °C bis 25 °C in Gegenwart eines Katalysators, wie beispielsweise eines Phosphins, und mindestens eines Lösungsmittels durchgeführt wird.

**6.** Verfahren nach Anspruch 1, wobei die reaktive Verbindung ein Keton ist, dadurch gekennzeichnet, daß die Umsetzung bei einer Temperatur im Bereich von 10 °C bis 50 °C und in Gegenwart mindestens eines kationischen Harzes in einem Mengenanteil von 20 bis 50 Gew.-%, bezogen auf die Epoxy(meth)acrylverbindung, erfolgt.

## Claims

**1.** Process for opening the epoxide function of a (meth)acrylic epoxide of general formula :

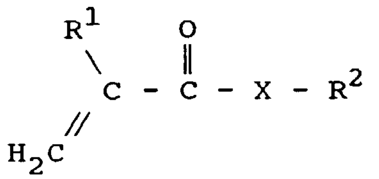

(I)

in which :

- X is chosen from oxygen and sulphur atoms, the NH radical, the radicals $NR^3$ in which $R^3$ is an alkyl group having from 1 to 12 carbon atoms, and the oxyalkylene radicals $O\text{-}(CH_2)_n$ in which n is an integer ranging from 3 to 16 ;
- $R^1$ is chosen from a hydrogen atom and alkyl radicals having from 1 to 5 carbon atoms ; and
- $R^2$ denotes a hydrocarbon chain comprising from 2 to 20 carbon atoms, chosen from straight-chain or branched alkyl, monocyclic or polycyclic cycloalkyl or heterocycloalkyl, and alkylaryl chains, the said hydrocarbon chain comprising an oxirane group in the chain, or at the end of the chain in the case of an alkyl or alkylaryl chain, or an exocyclic or endocyclic oxirane group in the case of a monocyclic or polycyclic cycloalkyl or heterocycloalkyl chain,
  it being also possible for $R^2$ to denote the glycidyl radical, the 2-epoxyethylbicyclo[2.2.1]hept-5(6)-yl radical or the epoxydicyclopentenyloxyethyl radical,

characterised by the fact that said (meth)acrylic epoxide is reacted with a compound chosen from :

- boron trifluoride etherate complexes, used in stoichiometric amount relative to the epoxide ;
- acid salts in the presence of the corresponding acid, used in excess in a ratio of 2 to 10 moles per mole of epoxide ;
- trialkylsilane or trialkoxysilane halides, used in a substantially stoichiometric amount relative to the epoxide ; and
- ketones in the presence of cationic resins.

**2.** Process according to claim 1, the reactive compound being a boron trifluoride etherate complex, characterised by the fact that the reaction takes place in the presence of a saturated alcohol having from 1 to 4 carbon atoms and at a temperature of between -50°C and +30°C.

3. Process according to claim 1, the reactive compound being an acid salt, characterised by the fact that the reaction is performed using the corresponding acid as solvent, at a temperature of between 20°C and 60°C.

4. Process according to claim 1, the reactive compound being an acid salt, characterised by the fact that said salt is sodium acetate or propionate, the corresponding acid being respectively acetic acid or propionic acid.

5. Process according to Claim 1, the reactive compound being a trialkylsilane halide or trialkoxysilane halide, characterised by the fact that said halide is used in a ratio of 1 mole to 1.33 mole per 1 mole of epoxide, the reaction being carried out in the presence of a catalyst, such as a phosphine, and of at least one solvent, at a temperature of from 0°C to 25°C.

6. Process according to Claim 1, the reactive compound being a ketone, characterised by the fact that the reaction takes place at a temperature of between 10°C to 50°C and in the presence of at least one cationic resin in an amount of 20 to 50% by weight relative to the (meth)acrylic epoxide.

Fig. 1

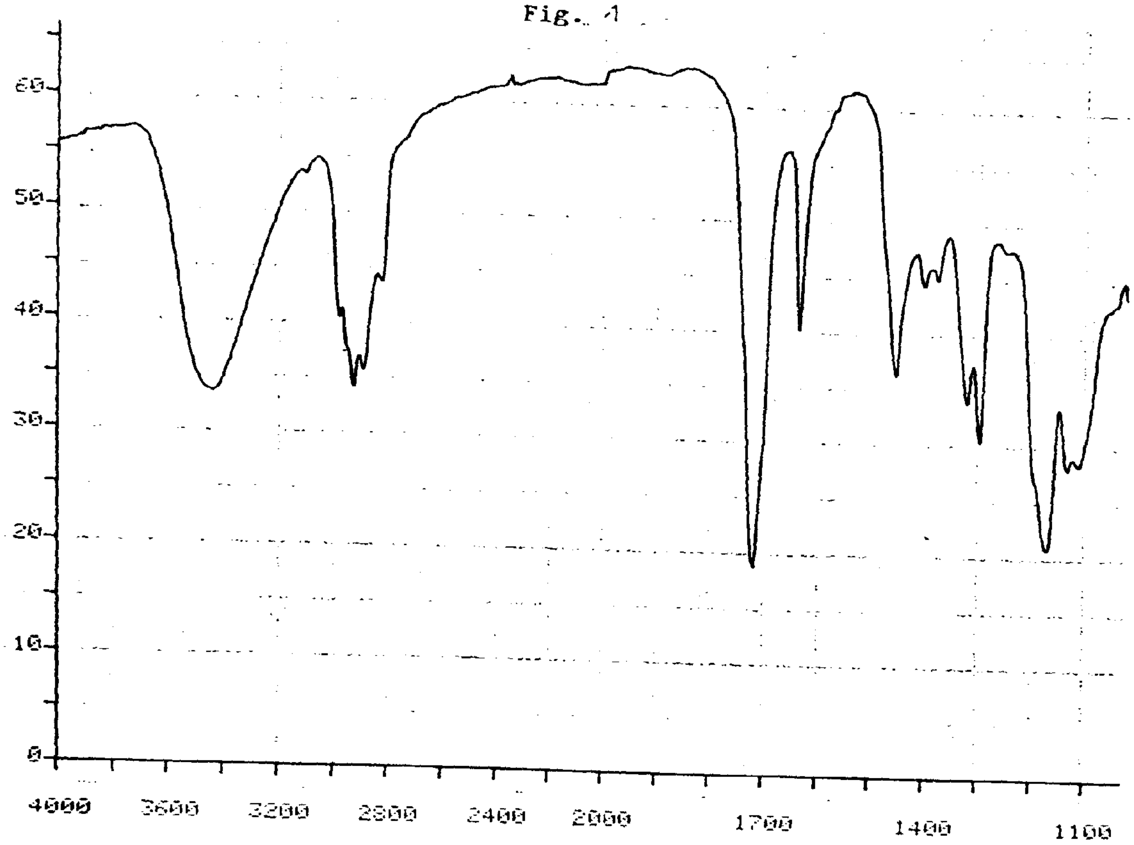
60
50
40
30
20
10
0
4000
3600
3200
2800
2400
2000
1700
1400
1100

Fig. 2

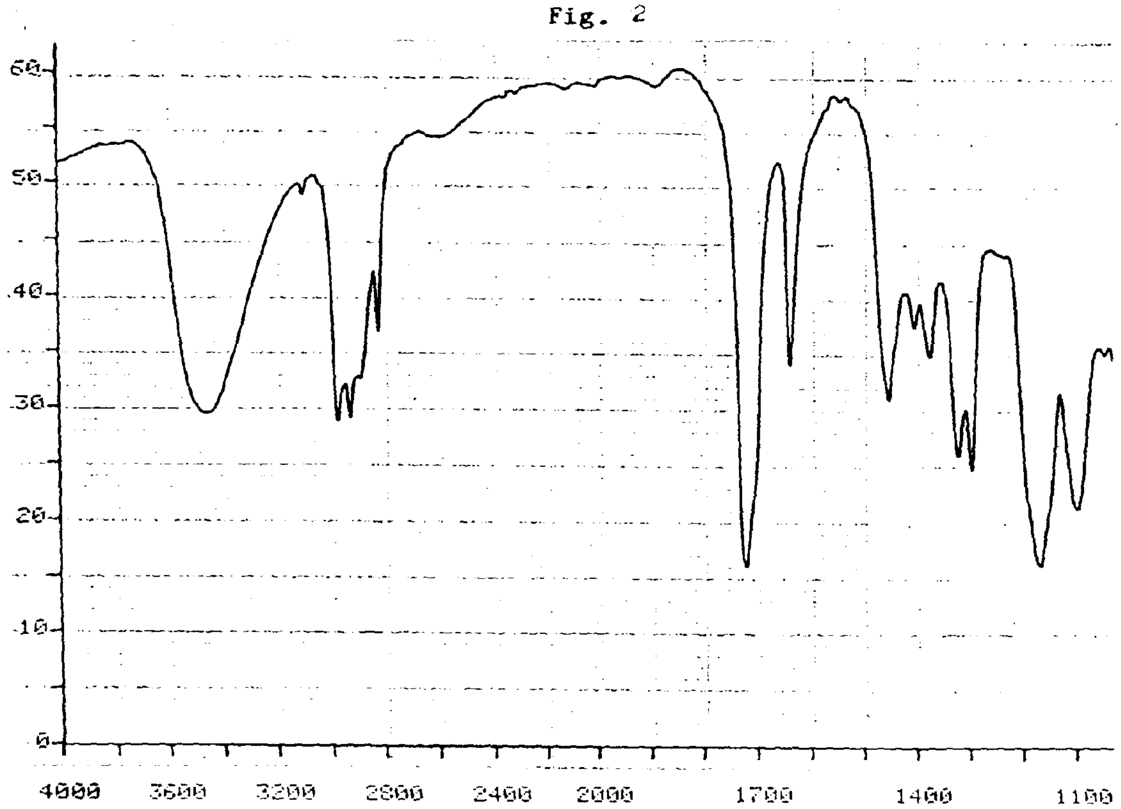
60
50
40
30
20
10
0
4000
3600
3200
2800
2400
2000
1700
1400
1100

Fig. 3

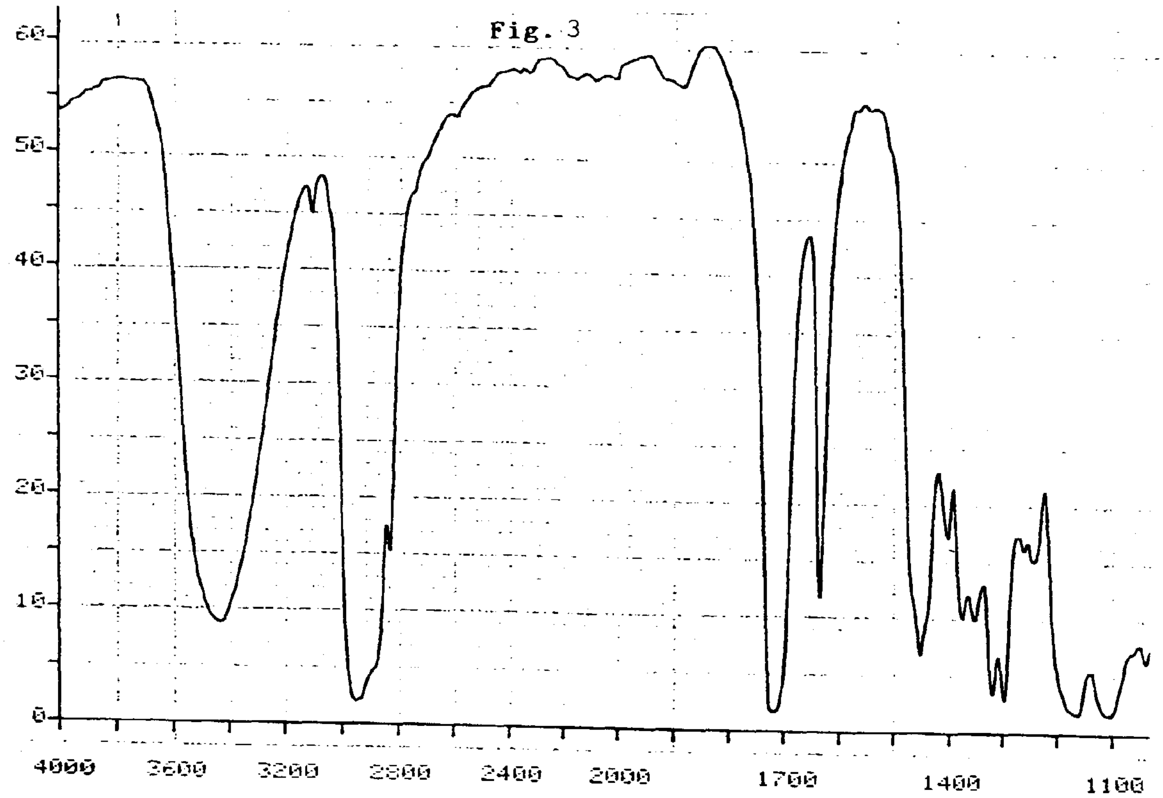
60
50
40
30
20
10
0
4000
3600
3200
2800
2400
2000
1700
1400
1100

Fig. 4

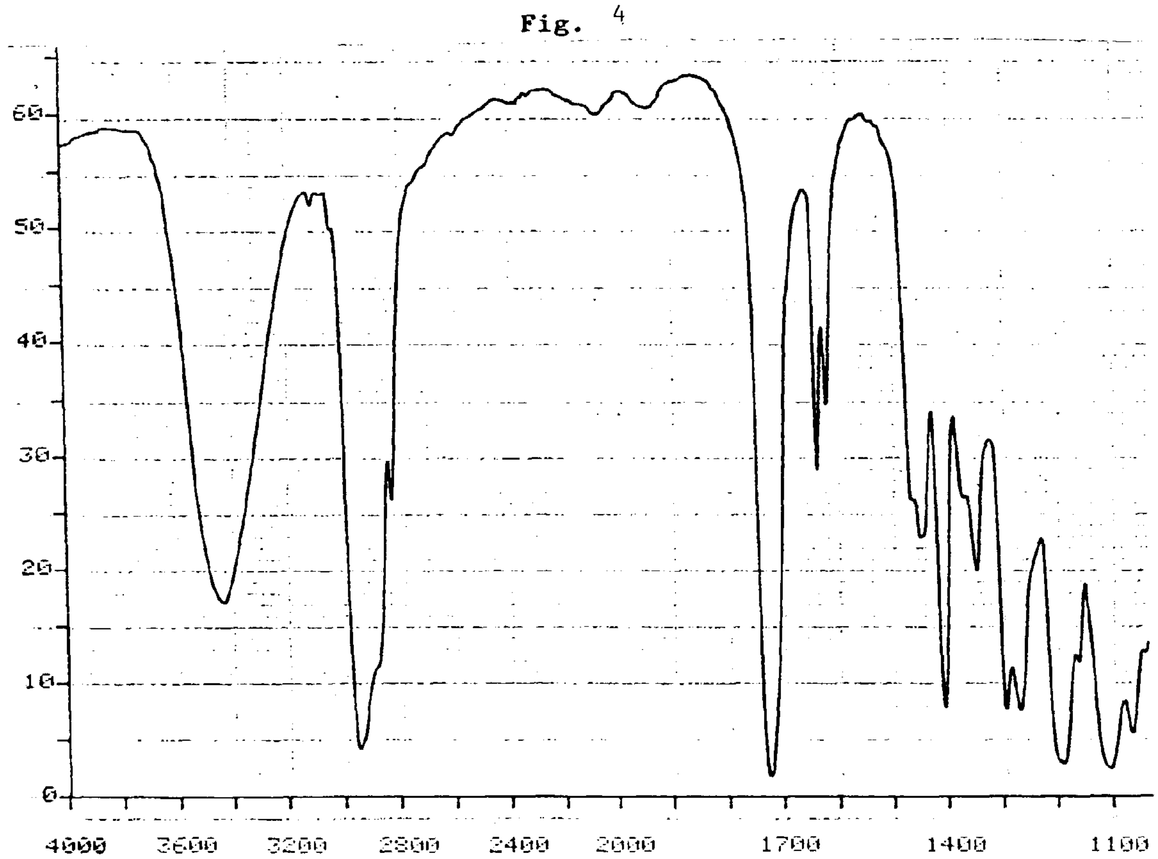
60
50
40
30
20
10
0
4000
3600
3200
2800
2400
2000
1700
1400
1100

Fig. 5

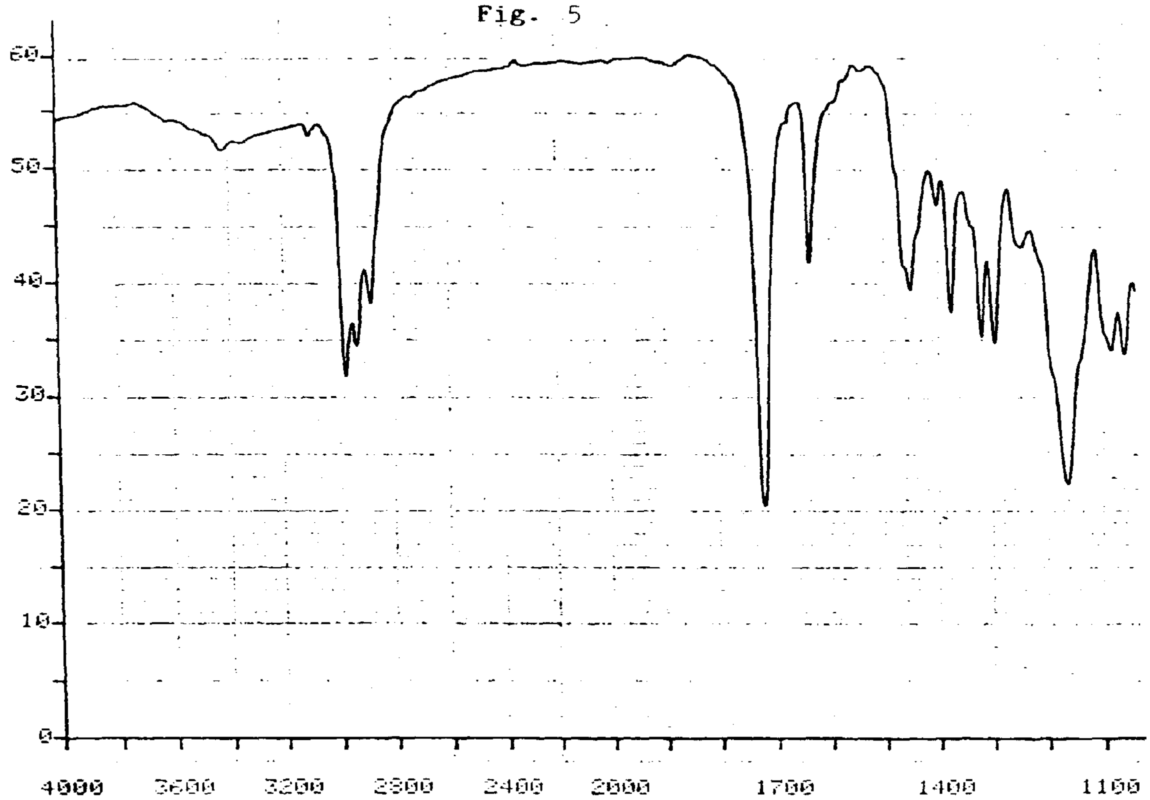
60
50
40
30
20
10
0
4000
3600
3200
2800
2400
2000
1700
1400
1100

Fig. 6

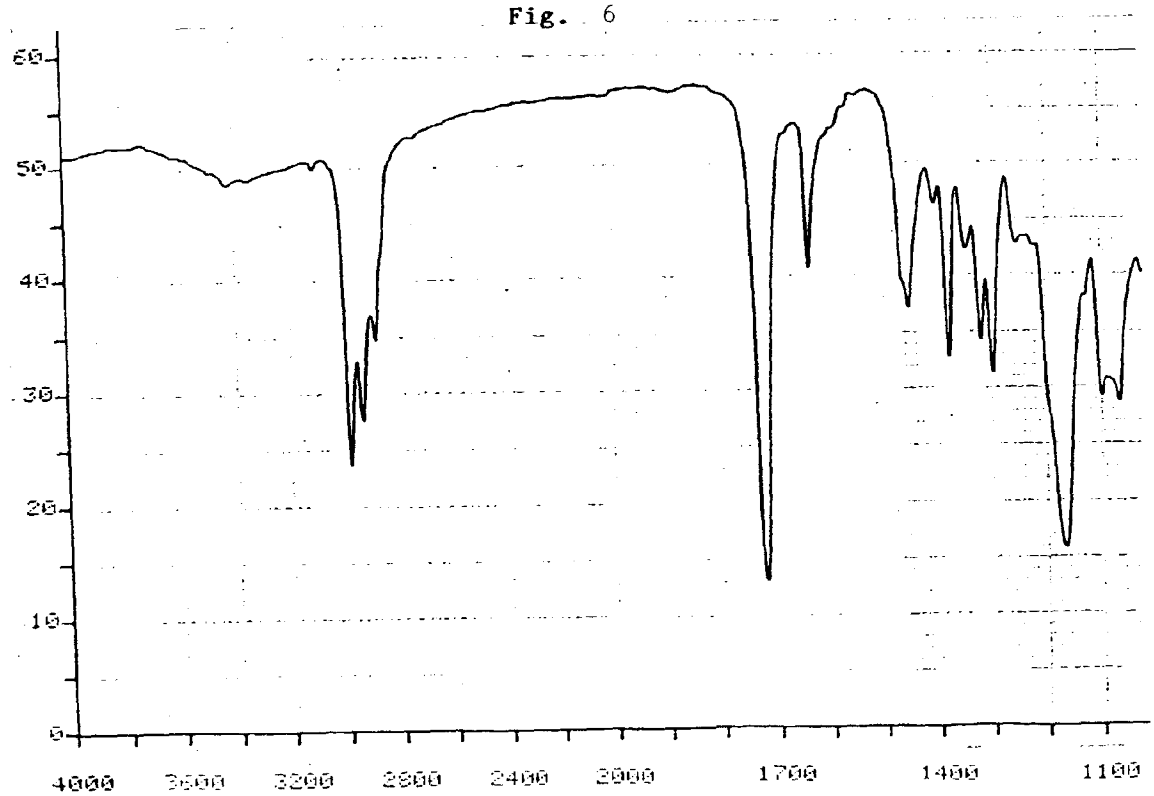
60
50
40
30
20
10
0
4000
3600
3200
2800
2400
2000
1700
1400
1100